# EUROPÄISCHE PATENTSCHRIFT

(11) **EP 0 530 446 B1**
(45) Veröffentlichungstag und Bekanntmachung des Hinweises auf die Patenterteilung: **28.01.1998**
(21) Anmeldenummer: 92108825.8
(22) Anmeldetag: 26.05.1992
(51) Int. Cl.: A61K 31/385, A61K 31/19

(54) **Verwendung von Schwefel enthaltenden Carbonsäuren zur Bekämpfung von pathophysiologisch bedingten Erregungsstörungen**
Use of sulfur containing carboxylic acids for combatting pathophysiologic excitatory disorders
Utilisation d'acides carboxyliques soufrés pour combattre les agents excicateurs d'origine physiopathologique

(30) Priorität: 05.07.1991 DE 4122303
(43) Veröffentlichungstag der Anmeldung: 10.03.1993
(73) Patentinhaber: ASTA Medica Aktiengesellschaft, D-01277 Dresden (DE)
(72) Erfinder: Ulrich, Heinz, Dr., W-8751 Niedernberg (DE); Hettche, Helmut, Dr., W-6957 Dietzenbach (DE); Weischer, Carl-Heinrich, Dr., W-5300 Bonn 1 (DE); Engel, Jürgen, Prof., W-8755 Alzenau (DE); Borbe, Harald, Dr., W-6500 Mainz 32 (DE); Dimpfel, W., Prof. Dr., W-6307 Linden 2 (DE)
(74) Vertreter: Decker, Karl-Ludwig

(56) Entgegenhaltungen:
- EP-A- 0 318 891
- EP-A- 0 427 246
- JOURNAL OF CEREBRAL BLOOD FLOW AND METABOLISM, Band 12, Nr. 1, 1992, Seiten 78- 87, Raven Press, Ltd, New York, US; J.H.M. PREHN et al.: "Dihydrolipoate reduces neuronal injury after cerebral ischemia"
- MED. WELT, Nr. 7, 18. Februar 1967, Seiten 395-400; K.H. BOYSEN: "Erfahrungen mit dem Präparat Thioctacid auf einer psychiatrischen Station"
- LEBER MAGEN DARM, Band 19, Nr. 4, Juli 1989, Seiten 212-213; P. FRÜHMORGEN: "Neue pharmakologische und klinische Erkenntnisse zum Einsatz der alpha- Liponsäure"
- DEVELOPMENTAL PHARMACOLOGY AND THERAPEUTICS, Band 14, Nr. 3, 1990, Seiten 193- 199, S. Karger AG, Basel, DE; W. DIMPFEL et al.: "Thioctic acid induces dose- dependent sprouting of neurites in cultured rat neuroblastoma cells"
- ARCHIVES OF PHARMACOLOGY, Band 339 (Suppl.), 1989, Seite R61; F. STROMAN et al. : "Cardioprotective properties of thioctic acid in anaesthetized rats and pigs"
- DIALOG FILE SUPPLIER, MEDLINE, AN=91297500; T.L. GAO et al.: "Effects of lipoic acid on reperfusion induced arrhythmias and myocardiac action potential alterations induced by free radiocal genarating system"
- DER NERVENARZT, Band 43, Nr. 5, Mai 1972, Seiten 266-267; L. BARTH: "Behandlung von akuten Zwischenfällen bei Rauschmittelgebrauch"
- J.E.F. REYNOLDS: "Martindale - The Extra Pharmacopoeia", 29. Auflage, 1989, Seite 1622, The Pharmaceutical Press, London, GB

## Beschreibung

Die Erfindung betrifft die Verwendung von Verbindungen der Formel 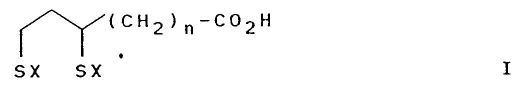 worin X ein Wasserstoffatom ist oder beide X eine einfache Bindung zwischen den beiden Schwefelatomen bedeuten und n eine Zahl von 1 bis 10 darstellt, beziehungsweise deren therapeutisch verwendbaren Salze zur Bekämpfung von Erregbarkeitsveränderungen infolge degenerativer Erkrankungen des Zentralnervensystems.

Aufgabe der Erfindung ist die Bereitstellung von verbesserten Arzneimitteln zur Bekämpfung von Erregbarkeitsveränderungen infolge degenerativer Erkrankungen des Zentralnervensystems.

Die in den Patentansprüchen und der Beschreibung angegebenen Gewichtsmengen beziehen sich jeweils auf die reinen Verbindungen der Formel I, beziehungsweise deren Enantiomeren, das heißt nicht auf die Salze.
Bei Verwendung von Salzen müssen die jeweils in Frage kommenden Dosismengen entsprechen und dem geänderten Molgewicht entsprechend erhöht werden.

Bei den Verbindungen der Formel I handelt es sich beispielsweise um α-Liponsäure (Racemat und die beiden optischen Isomeren R(+)- und S(-)-Form) sowie um die Dihydroliponsäure (Racemat und die beiden optischen Isomeren R(+)- und S(-)-Form), das heißt Verbindungen der Formel I, worin n die Zahl 4 ist und X die angegebenen Bedeutungen hat.
Besonders wirksam hinsichtlich der in vorliegender Patentanmeldung beschriebenen Wirkungen ist die α-Liponsäure beziehungsweise die S(-)-Form der α-Liponsäure. Insbesondere gilt dies für die Wirkung bei Entzugssymptomatik und Rauschmittelabusus.

α-Liponsäure ist in Form des Racemats (Thioctsäure®) in Pflanzen und Tieren weit verbreitet; sie wirkt in vielen enzymatischen Reaktionen als Coenzym, stellt einen Wachstumsfaktor für manche Bakterien und Protozoen dar und wird bei Knollenblätterpilzvergiftungen eingesetzt. Weiterhin weist das α-Liponsäure-Racemat antiphlogistische, antinociceptive (analgetische) sowie zytoprotektive Eigenschaften auf.

Thioctsäure ist als Arzneimittel für folgende Indikationen auf dem Markt:
Fettleber und Fettzirrhose,
besonders durch Alkohol chronische Lebererkrankung,
durch Pilzvergiftung verursachte Leberschädigung,
diabetische Neuropathie,
alkoholische Neuropathie.

Dihydroliponsäure ist die 6,8-Dimercapto-octansäure. Aus Tieruntersuchungen ist bekannt, daß Dihydroliponsäure Schlangengift inaktiviert. Diese Untersuchungen erfolgten beispielsweise an Ratten und Mäusen, wobei Lösungen in Wasser oder physiologischen Kochsalzlösung verwendet wurden, die das Schlangengift und Dihydroliponsäure enthielten.

Die Verbindungen der Formel I können auch in Form ihrer therapeutisch verwendbaren Salze eingesetzt werden. Die Herstellung solcher Salze erfolgt in hierfür bekannter Weise.
Als Salzbildner kommen zum Beispiel übliche Basen beziehungsweise Kationen in Frage, die in der Salzform physiologisch verträglich sind. Beispiele hierfür sind:
Alkali- oder Erdalkalimetalle, Ammoniumhydroxid, basische Aminosäuren wie Arginin und Lysin, Amine der Formel NR₁R₂R₃ worin die Reste R₁, R₂ und R₃ gleich oder verschieden sind und Wasserstoff, C₁-C₄-Alkyl oder C₁-C₄-Oxyalkyl bedeuten wie Mono- und Diethanolamin, 1-Amino-2-propanol, 3-Amino-1-propanol; Alkylendiamine mit einer Alkylenkette aus 2 bis 6-C-Atomen wie Ethylendiamin oder Hexamethylentetramin, gesättigte cyclische Aminoverbindungen mit 4 bis 6 Ringkohlenstoffatomen, wie Piperidin, Piperazin, Pyrrolidin, Morpholin; N-Methylglucamin, Kreatin, Trometamol.

Die erfindungsgemäßen Verbindungen der Formel I besitzen eine günstige Wirkung bei Erregbarkeitsveränderungen infolge degenerativer (vorzugsweise neurodegenerativer) Erkrankungen des Zentralnervensystems. Insbesondere eignen sich die erfindungsgemäßen Verbindungen auch zur Bekämpfung von Entzugssymptomen wie sie nach Abusus von Alkohol, Beruhigungsmitteln oder Rauschgift auftreten. Entsprechende Arzneimittel, die die Verbindungen der Formel I als Wirkstoffe enthalten, besitzen beispielsweise eine antidegenerative und antiatrophisierende sowie erregungsstabilisierende Wirkung auf das Zentralnervensystem.
Weiterhin sind die Verbindungen der Formel I auf Grund ihrer erregungsstabilisierenden Wirkung in der Lage, auch funktionelle Störungen des Zentralnervensystems günstig zu beeinflussen. Beispielhaft seien hier Fntzugssymptomatik nach Rauschmittelabusus beziehungsweise das Krankheitsbild der "Panic Disorder" genannt.
Ebenfalls zeigen die Verbindungen der Formel I auch eine Wirkung bei Schädigungen des Hirns, zum Beispiel bei Morbus Alzheimer, Korsakow Syndrom insbesondere im Hinblick auf die mit diesen Krankheiten verbundenen Gedächtnisstörungen, wie sie auch im Rahmen des hirnorganischen Psychosyndroms auftreten.

Die erfindungsgemäßen Verbindungen der Formel I zeigen beispielsweise im Modell zum Nachweis der Prevention der durch hypoglykämischen beziehungsweise ischämischen Insult verursachte Veränderungen im EEG der Ratte (im Vergleich zum Vorwert) bei der parenteralen Applikation (i.p.=intraperitoneal) bei 30 mg/kg (i.p.) des Racemats der alpha-Liponsäure 118 %, bei 30 mg/kg i.p. des (+)-Enantiomers 157 % und bei 30 mg/kg i.p. des (-)-Enantiomers 85 % Änderung der Leistungsdichte des beta-2-Bandes im frontalen Kortex.
Der Dosisbereich für eine Wirkung in dem oben genannten Versuchsmodell beträgt beispielsweise 10 - 100 mg/kg insbesondere 15 - 45 mg/kg für die parenterale (intraperitoneale) Applikation.

Diese Untersuchung erfolgte analog der Methode Dimpfel et al, Neuropsychobiology 16: 163 - 168, (1986) mit der Änderung, daß die Ableitung vom Hippokampus anstelle des Thalamus erfolgt.
(Radioelectroencephalography (Tele-Stereo-EEG) in the rat as a pharmacological model to differentiate the central action of flupirtine from that of opiates, diazepam and phenobarbital)
Dieser Test ist ein Nachweis für eine Wirkung bei folgenden Krankheitsbildern:
Allergische und Stoffwechselstörungs-bedingte zentrale und periphere Neuropathien mit Störungen der Gedächtnisleistung sowie der Nervenleitgeschwindigkeit zum Beispiel altersbedingtes Nachlassen der Hirnleistung (Minor brain dysfunction).
Kurze Beschreibung der zuvor erwähnten Testmethode:
Männliche Fischer-344 Ratten wurden im Alter von 4 bis 5 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Pro Science Computer System in Echtzeit einer Fast Fourier Transformation unterworfen und die Leistungsdichtespektren jeweils über 15 Minuten gemittelt. Die Unterteilung der Spektren in 6 verschiedene Frequenzbereiche erlaubte die Erfassung pharmakonspezifischer Veränderungen in Bezug auf die jeweils vor der Applikation gemessenen Vorwerte innerhalb dieser Frequenzbänder.

Zum Applikationsprotokoll der Substanzen: Die Substanzen wurden intraperitoneal 45 Minuten nach Beginn der Messungen (Vorwert) injiziert. Fünf Minuten später wurden die Messungen wieder gestartet, mindestens über die nächsten 2 Stunden kontinuierlich analysiert und in 15-minütigen Perioden zusammengefaßt. Die Testsubstanzen (zum Beispiel Thioctsäure) wurden in einer Dosierung von 30 mg/kg appliziert.

Die experimentelle Serie wurde mit der Injektion von Placebo begonnen. Darauf folgte die erste Prüfung von Deprenyl. Nach 4 Wochen wurde beispielsweise mit der chronischen Applikation von R(+)-α-Liponsäure begonnen und über 7 Tage bei 2 Injektionen/Tag aufrechterhalten. Zwei Tage nach der letzten Injektion wurde wiederum die Wirkung von Deprenyl gemessen. Nach einer Pause für die Tiere von einer Woche wurde nochmals die Wirkung von R(+)-α-Liponsäure überprüft, desweiteren wiederum im Abstand von einer Woche. Nach der darauf folgenden Auswaschphase von einer weiteren Woche und einer weiteren Placeboserie wurde mit der chronischen Applikation von S(-)-α-Liponsäure begonnen (insgesamt 4 Wochen nach Beendigung der ersten chronischen Applikation). Zum Abschluß wurde 2 Tage nach der letzten S-(-)-α-Liponsäure-Applikation noch einmal die Wirkung von Deprenyl erfaßt. Während der chronischen Applikation von α-Liponsäure (Racemat) wurde am 1., 3., 5. und 7. Tag die Wirkung gemessen.

Der statistische Vergleich der Versuche erfolgte mit Hilfe einer multivarianten Analyse nach Ahrens und Läuter (1974) auf der Basis der Veränderungen innerhalb der einzelnen Frequenzbänder in allen Hirnregionen als Variablen.

Als Indikationen für die Verbindungen der Formel I kommen zum Beispiel in Betracht:
Gewebeschädigende Prozesse des Zentralnervensystems und des Herzens, sowie die daraus resultierenden Gewebsnekrosen; ischämisch oder hypoxisch bedingte Organspätschäden, insbesondere des Zentralnervensystems und des Herzens wie zum Beispiel Mikro- und Makroangiopathien und die damit verbundenen Überregbarkeitsphänomene; Rhythmusstörungen des Herzens; Schädigungen von Hirn und Herz zum Beispiel Morbus Alzheimer, Korsakow Syndrom insbesondere im Hinblick auf die mit diesen Krankheiten verbundenen Gedächtnisstörungen wie sie auch im Rahmen des hirnorganischen Psychosyndroms auftreten; allergische und stoffwechselstörungsbedingte (zum Beispiel durch Diabetes) zentrale und periphere Neuropathien und Polyneuropathien (mit Störungen der Gedächtnisleistung sowie der Nervenleitgeschwindigkeit); altersbedingtes Nachlassen der Hirnleistung (Minor brain dysfunction). Neuropathophysiologische Entgleisungen der zentralen Erregbarkeit wie im Krankheitsbild der "Panic Disorder" (Margraf J. und Schneider S.: Panik, erschienen im Springer Verlag 1990) sowie Entzugssymptomatik nach Rauschmittelabusus.

Belegt wird die zuletzt genannte Indikation beispielsweise durch Versuche an Ratten, die über 2 Wochen täglich mit höheren Dosen von Alkohol oder Morphin behandelt werden. Die Gehirne dieser Tiere zeigen nach 16-stündigem Entzug eine in vitro unter kontrollierten Bedingungen sauber erfaßbare Übererregbarkeit, die durch Gegenwart von 100µM einer Verbindung der Formel I vollständig normalisiert wird. Eine ausführliche Beschreibung dieser Versuche ist der Anhang A. Dieser Anhang A ist Bestandteil der Beschreibung dieser Patentanmeldung.

Die Tagesdosen der erfindungsgemäßen Darreichungsformen für die oben genannten Wirkungen bestehen zum Beispiel aus 0,1 bis 800 mg, vorzugsweise 15 bis 400 mg und insbesondere 50 bis 300 mg Verbindung I (zum Beispiel α-Liponsäure oder Dihydroliponsäure oder deren Enantiomeren).

Erfindungsgemäß wird eine Tagesdosis an Verbindung I (zum Beispiel α-Liponsäure oder der Dihydroliponsäure oder deren Enantiomeren) von 10 bis 800 mg, beispielsweise von 25 bis 400 mg, insbesondere 10 bis 300 mg verabreicht.

Die maximale Tagesdosis soll 800 mg nicht überschreiten. Die Tagesdosen können in Form einer einmaligen Verabreichung der gesamten Menge oder in Form von 1 bis 6, insbesondere 1 bis 4 Teildosen pro Tag eingesetzt werden. Im allgemeinen ist eine Verabreichung von 1 bis 4 mal, insbesondere 1 bis 3 mal täglich bevorzugt. Beispielsweise beträgt die bevorzugte Tagesdosis 80 bis 350 mg für die parenterale Applikationsform und 800 mg für die orale Form. Insbesondere beträgt die Tagesdosis für die parenterale Applikationsform 50 mg und 400 mg für die orale Form.

Vorzugsweise werden die Arzneimittel oral verabreicht.

Die Verbindungen der Formel I (zum Beispiel α-Liponsäure und die Dihydroliponsäure oder deren Enantiomere) können auch in Form einer Lösung appliziert werden, beispielsweise peroral, topisch, parenteral, intravenös, intramuskulär, subkutan, nasal, inhalativ, rektal, transdermal.

Die Arzneimittel, die als Wirkstoff eine oder mehrere Verbindungen der Formel I (zum Beispiel α-Liponsäure oder die Dihydroliponsäure oder deren Enantiomere) enthalten, können zum Beispiel in Form von Tabletten, Kapseln, Pillen oder Dragees, Granulaten, Suppositorien, Pellets, Lösungen oder Emulsionen formuliert werden, wobei der Wirkstoff mit entsprechenden Hilfs- und Trägerstoffen kombiniert wird. Lösungen enthalten beispielsweise 0,5 bis 20 Gewichts%, vorzugsweise 1 bis 10 Gewichts% Verbindung der Formel I.

Die Dosierungseinheit der Arzneimittel, die eine oder mehrere Verbindungen der Formel I oder ein therapeutisch verwendbares Salz hiervon als Wirkstoff enthalten, kann beispielsweise enthalten:
a) bei peroralen Arzneiformen:
   insbesondere 50 bis 400 mg Verbindung/Wirkstoff I (zum Beispiel α-Liponsäure oder Dihydroliponsäure). Die Dosen können beispielsweise 1 bis 6, vorzugsweise 1 bis 4, insbesondere 1 bis 3 mal täglich verabreicht werden. Jedoch soll eine Gesamtdosis von 800 mg pro Tag nicht überschritten werden. Dasselbe gilt auch für die folgenden unter b) bis e) aufgeführten Arzneiformen.
b) bei parenteralen Arzneiformen (zum Beispiel intravenös, intramuskulär):
   10 bis 350 mg, vorzugsweise 15 bis 350 mg, insbesondere 20 bis 150 mg Verbindung/Wirkstoff I. Die Dosen können beispielsweise 1 bis 6, vorzugsweise 1 bis 4, insbesondere 1 bis 3 mal täglich verabreicht werden.
c) bei Arzneiformen zur rektalen Applikation:
   10 bis 500 mg, vorzugsweise 40 bis 400, insbesondere 50 bis 200 mg Verbindung/Wirkstoff I. Diese Dosen können beispielsweise 1- bis 6, vorzugsweise 1- bis 4, insbesondere 1- bis 3 mal täglich verabreicht werden.
d) bei Arzneiformen zur Applikation auf die Haut und Schleimhäute (zum Beispiel als Lösungen, Lotionen, Emulsionen, Salben und so weiter):
   10 bis 500 mg Verbindung/Wirkstoff I, vorzugsweise 40 bis 250 mg, insbesondere 5 bis 200 mg. Die Dosen können beispielsweise 1 bis 6, vorzugsweise 1 bis 4, insbesondere 1 bis 3 mal täglich verabreicht werden.
e) Bei Arzneiformen zur Inhalation von Lösungen oder Aerosolen:
   0,1 bis 300 mg, vorzugsweise 0,25 bis 150 mg, insbesondere 0,5 bis 80 mg Verbindung/Wirkstoff I. Diese Dosen können beispielsweise 1 bis 6, vorzugsweise 1 bis 4, insbesondere 1 bis 3 mal täglich verabreicht werden.
   Falls Lösungen verwendet werden, wird die Verbindung/Wirkstoff I vorzugsweise in Form eines Salzes eingesetzt.

Selbstverständlich können auch galenische Zubereitungen hergestellt werden, welche die oben angegebenen Dosierungseinheiten 2 bis beispielsweise 6 mal enthalten.

Insbesondere enthalten Tabletten oder Kapseln 20 bis 600 mg; Pellets, Pulver oder Granulate 20 bis 500 mg; Suppositorien 20 bis 400 mg an Verbindung/Wirkstoff I.

Die Arzneimittel mit einem oder mehreren Wirkstoffen der Formel I enthalten zweckmäßig auch 0,001 bis 1 Gewichtsteile Antioxydans (bezogen auf 1 Gewichtsteil Verbindung I).
Als Antioxydantien kommen beispielsweise in Betracht: Natriumsulfit, Natriumhydrogensulfit, Natriummetabisulfit, Ascorbinsäure, Ascorbylpalmitat, -myristat, -stearat, Gallussäure, Gallussäure-alkylester, Butylhydroxyanisol, Nordihydroguajaretsäure, Tocopherole sowie Synergisten (Stoffe, die Schwermetalle durch Komplexbildung binden, beispielsweise Lecithin, Ascorbinsäure, Phosphorsäure, Ethylendiaminotetraessigsäure, Citrate, Tartrate) zur Anwendung. Der Zusatz der Synergisten steigert die antioxygene Wirkung der Antioxydanten erheblich.

Als Konservierungsmittel für die erfindungsgemäßen Arzneimittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, Chlorhexidin und Formalinderivate.

Als Komplexbildner kommen beispielsweise in Frage: Chelatbildner wie Ethylendiamino-tetraessigsäure, Nitrilotriessigsäure, Diethylentriaminpentaessigsäure sowie deren Salze.
Weiterhin kommen als Komplexbildner auch solche in Frage, die Dihydroliponsäure in einem Hohlraum einschließen. Beispiele hierfür sind Harnstoff, Thioharnstoff, Cyclodextrine, Amylose.

Gegebenenfalls ist zur Stabilisierung des Wirkstoffmoleküls mit physiologisch verträglichen Basen oder Puffern auf einen pH-Bereich von ca. 6 bis 9 einzustellen. Im allgemeinen wird ein möglichst neutraler bis schwach basischer (bis pH 8) pH-Wert bevorzugt.

### Beispiele für pharmazeutische Zubereitungen

### Beispiel 1:

### Tabletten mit 50 mg S- bzw. R-α-Liponsäure

250 g S-α-Liponsäure werden mit 750 g mikrokristalliner Cellulose gleichmäßig verrieben. Nach dem Sieben der Mischung werden 250 g Stärke (Starch 1500/ Colorcon), 732,5 g Lactose, 15 g Magnesiumstearat und 2,5 g hochdisperses Siliciumdioxid zugemischt und die Mischung zu Tabletten vom Gewicht 400,0 mg verpreßt.

Eine Tablette enthält 50 mg S-α-Liponsäure.

In gleicher Weise können Tabletten mit 50 mg R-α-Liponsäure hergestellt werden, wenn statt 250 g S-α-Liponsäure die gleiche Menge R-α-Liponsäure eingesetzt wird.

Gegebenenfalls können die Tabletten nach üblichen Verfahren mit einem magensaftlöslichen oder magensaftpermeablen Filmüberzug versehen werden.

### Beispiel 2:

### Ampullen mit 50 mg S- bzw. R-α-Liponsäure als Trometamolsalz in 2 ml

250 g S-α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 µm mit Glasfaservorfilter filtriert. Das Filtrat wird unter aseptischen Bedingungen zu 2 ml in sterilisierte 2 ml-Ampullen abgefüllt.

Eine Ampulle enthält in 2 ml Injektionslösung 50 mg S-α-Liponsäure als Trometamolsalz.

In gleicher Weise können Ampullen mit R-α-Liponsäure hergestellt werden, wenn statt 250 g S-α-Liponsäure die gleiche Menge R-α-Liponsäure eingesetzt wird.

### Beispiel 3

### Ampullen mit 250 mg Dihydroliponsäure in 10 ml Injektionslösung

60 g Trometamol und 1 g Ethylendiamintetraessigsäure, Dinatriumsalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g Dihydroliponsäure gelöst. Die Lösung wird mit Wasser für Injektionszwecke, das mit Stickstoff begast wurde, auf ein Volumen von 2 Liter aufgefüllt. Nach sorgfältigem Mischen wird die Lösung über ein Membranfilter der Porenweite 0,2 µm filtriert und das Filtrat unter aseptischen Bedingungen und unter Vor- und Nachbegasung mit Stickstoff in Ampullen zu 10 ml Füllvolumen abgefüllt.

Eine Ampulle enthält in 10 ml Lösung 250 mg Dihydroliponsäure als Trometamolsalz.

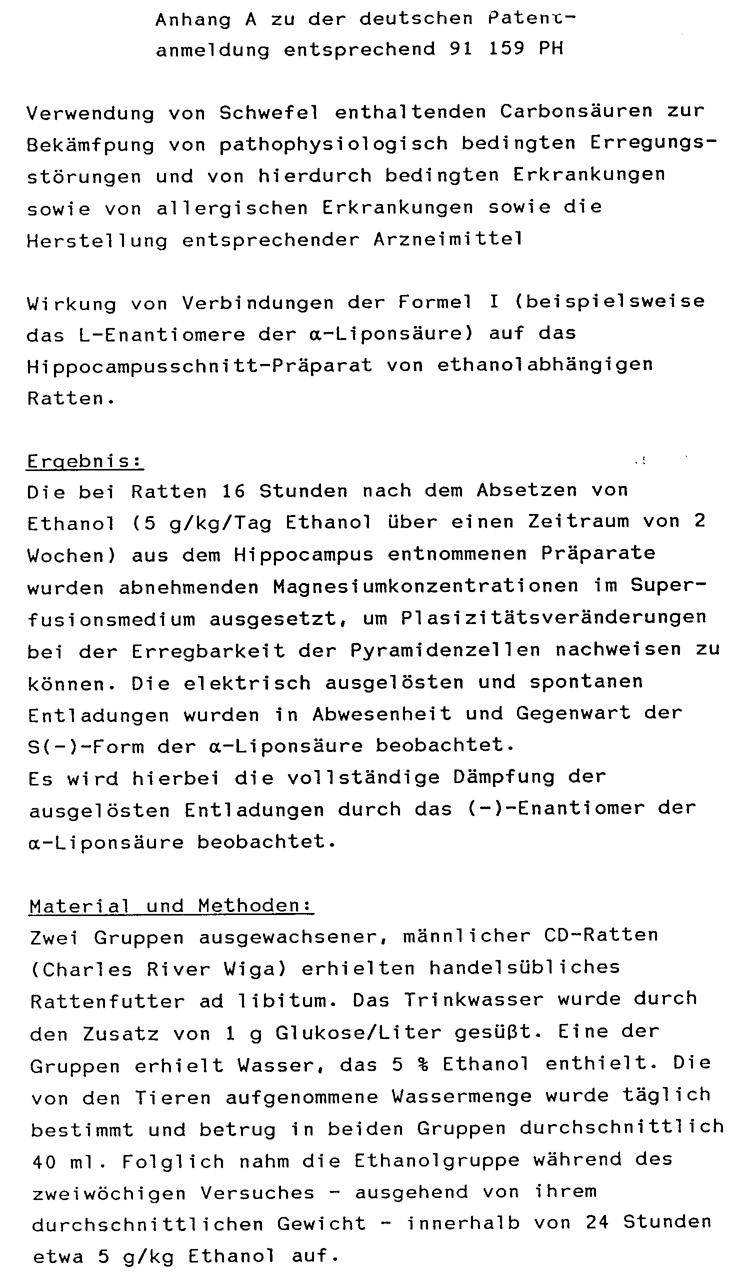

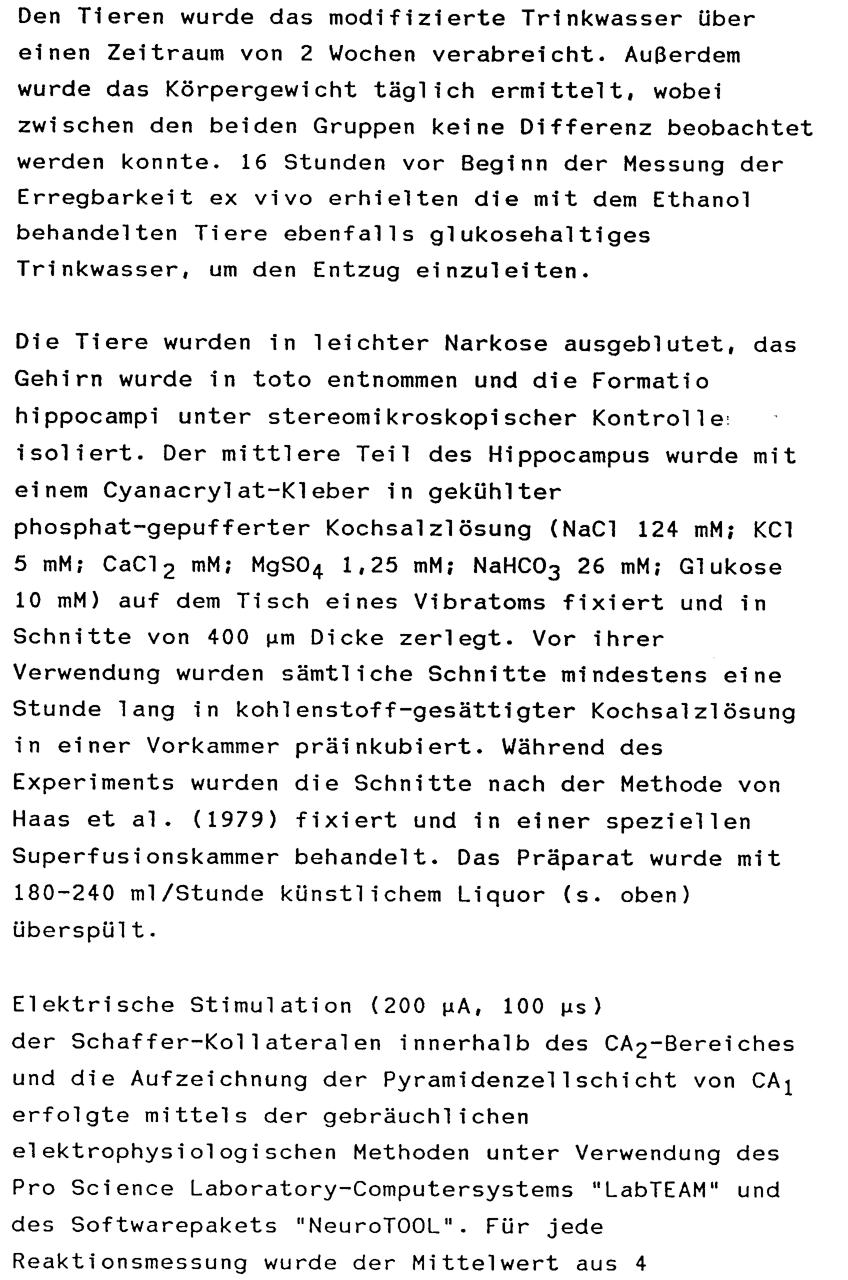

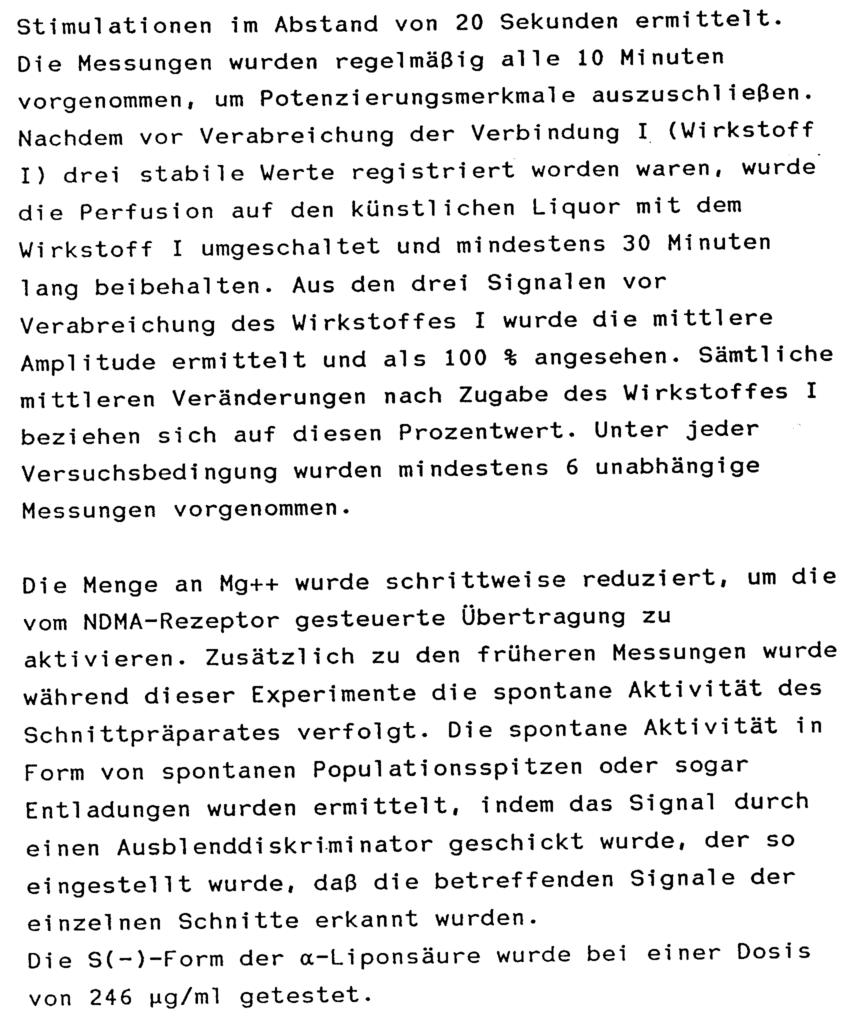

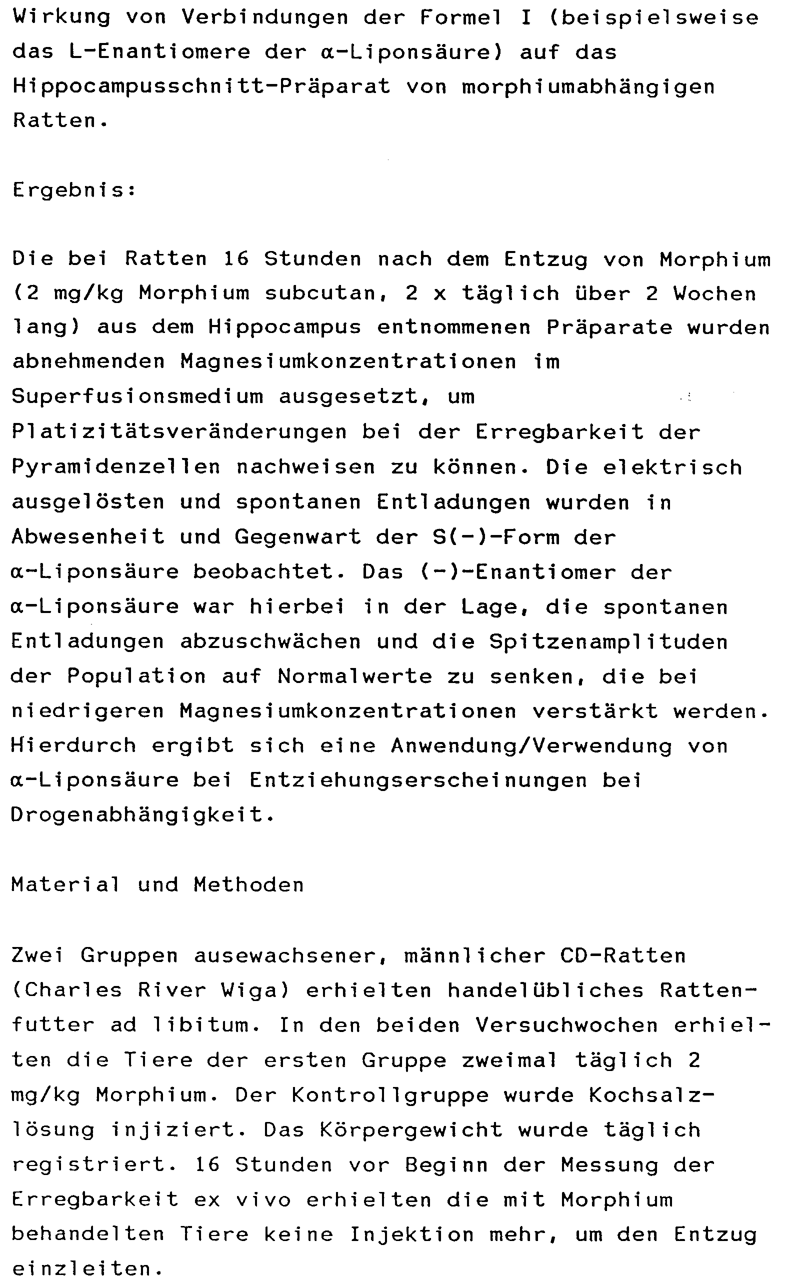

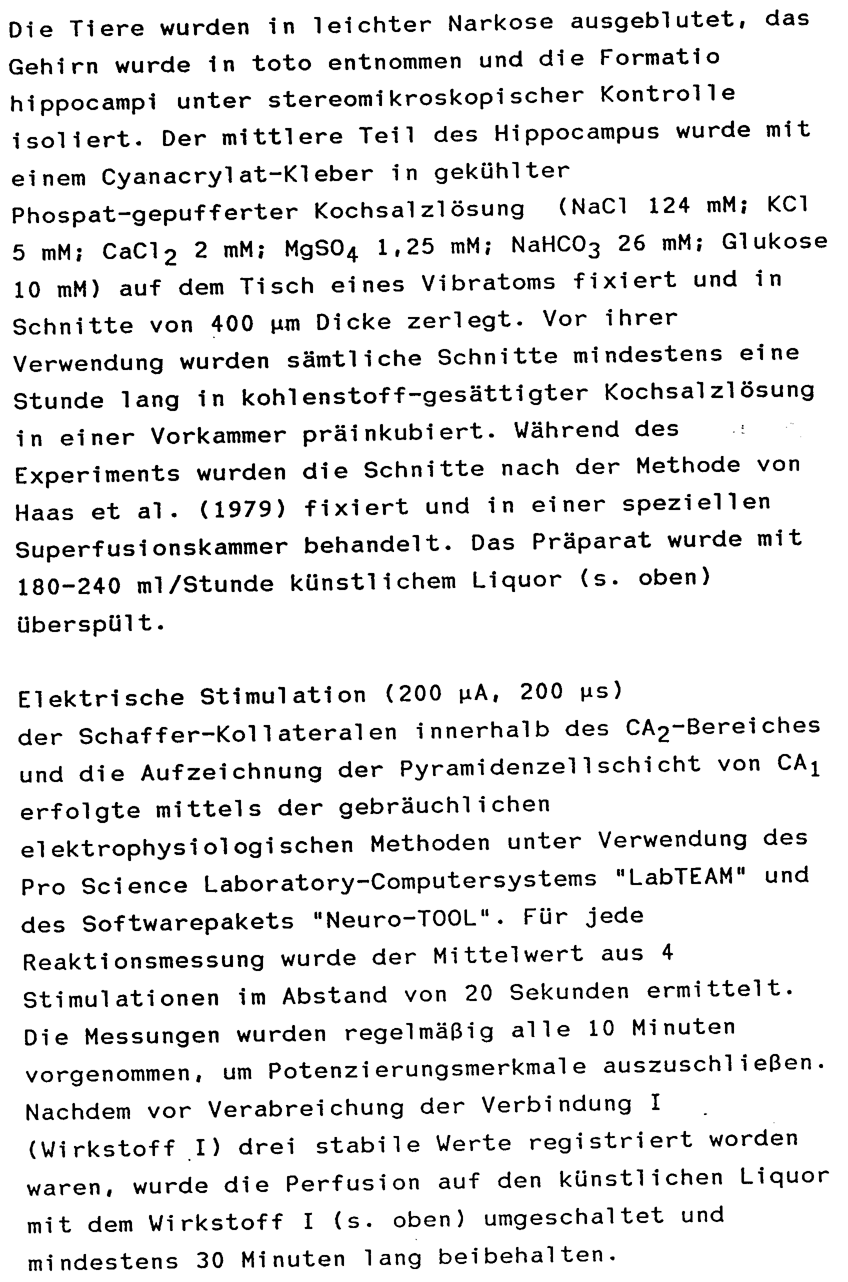

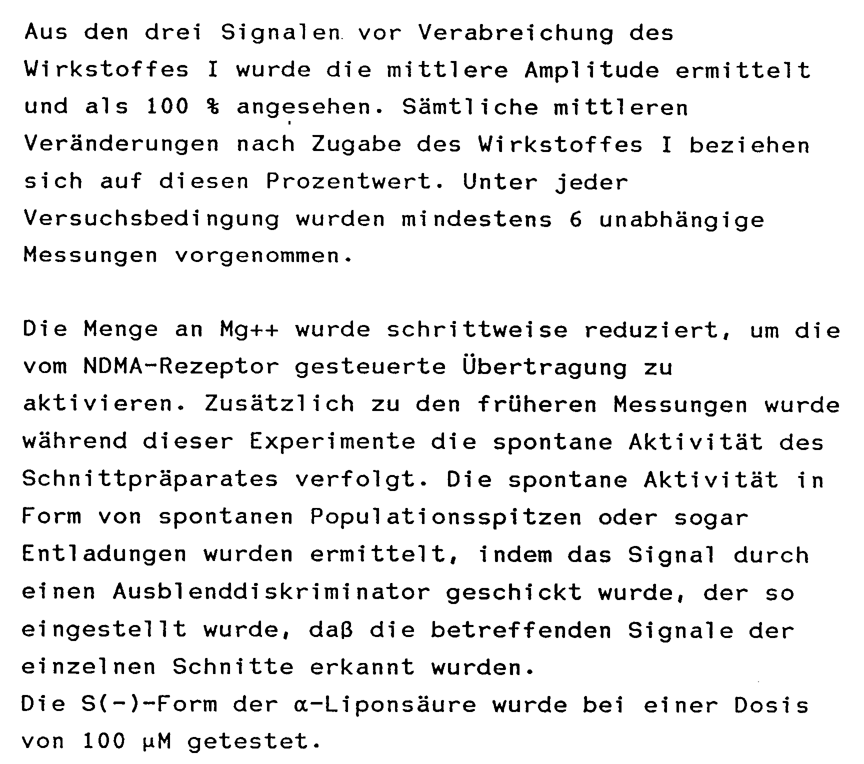

## Patentansprüche

1. Verwendung von Verbindungen der Formel 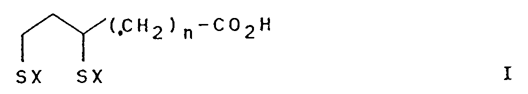 worin X ein Wasserstoffatom ist oder beide X eine einfache Bindung zwischen den beiden Schwefelatomen bedeuten und n eine Zahl von 1 bis 10 darstellt oder deren therapeutisch verwertbaren Salzen für die Herstellung eines Arzneimittels zur Bekämpfung von Erregbarkeitsveränderungen infolge degenerativer Erkrankungen des Zentralnervensystems.

2. Verwendung nach Anspruch 2, dadurch gekennzeichnet, daß das Arzneimittel übliche pharmazeutische Träger-, Hilfs- und/oder Verdünnungsmittel enthält.

3. Verwendung nach einem der vorangegangenen Ansprüche, dadurch gekennzeichnet, daß das Arzneimittel ein pharmazeutisch verträgliches Antioxidans und/oder einen pharmazeutisch verträglichen Komplexbildner enthält.

4. Verwendung nach einem der vorangegangenen Ansprüche, dadurch gekennzeichnet, daß das Arzneimittel die Wirkstoffe der Formel I in einer Menge von 0,1 bis 800 mg enthält.

## Claims

1. Use of compounds of the formula 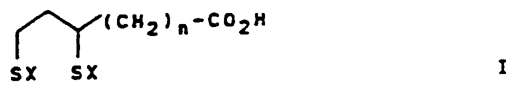 in which X is a hydrogen atom or both Xs are a single bond between the two sulphur atoms and n is a number from 1 to 10, or their therapeutically utilizable salts for the production of a medicament for the control of excitability changes as a result of degenerative disorders of the central nervous system.

2. Use according to Claim 2, characterized in that the medicament contains customary pharmaceutical excipients, auxiliaries and/or diluents.

3. Use according to one of the preceding claims, characterized in that the medicament contains a pharmaceutically tolerable antioxidant and/or a pharmaceutically tolerable complexing agent.

4. Use according to one of the preceding claims, characterized in that the medicament contains the active compounds of the formula I in an amount from 0.1 to 800 mg.

## Revendications

1. Utilisation de composés de formule 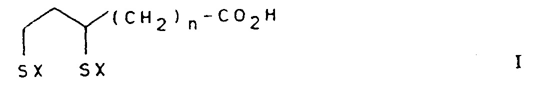 dans laquelle X représente un atome d'hydrogène ou les deux X représentent une liaison simple entre les deux atomes de soufre et n représente un nombre de 1 à 10, ou de leurs sels utilisables du point de vue thérapeutique pour la préparation d'un médicament pour la lutte contre des maladies dégénératives du système nerveux central par suite de modifications de l'excitabilité.

2. Utilisation selon la revendication 2, caractérisée en ce que le médicament contient des supports, auxiliaires et/ou diluants pharmaceutiques usuels.

3. Utilisation selon l'une quelconque des revendications précédentes caractérisée en ce que le médicament contient un antioxydant compatible du point de vue phamaceutique et/ou un agent complexant compatible du point de vue pharmaceutique.

4. Utilisation selon l'une quelconque des revendications précédentes, caractérisée en ce que le médicament contient les substances actives de formule I en une quantité de 0,1 à 800 mg.
